# EUROPEAN PATENT SPECIFICATION

(11) **EP 3 501 373 B1**
(45) Date of publication and mention of the grant of the patent: **17.01.2024**
(21) Application number: 19154626.6
(22) Date of filing: 25.02.2013
(51) Int. Cl.: A61B 1/00, A61B 1/012

(54) **DISTAL PORTION OF RIGID ENDOSCOPE AND METHOD OF APPLYING ILLUMINATION FIBERS**
DISTALER ABSCHNITT EINES STARREN ENDOSKOPS UND VERFAHREN ZUR AUFTRAGUNG VON BELEUCHTUNGSFASERN
PARTIE DISTALE DE L'ENDOSCOPE RIGIDE ET PROCÉDÉ D'APPLICATION DE FIBRES D'ÉCLAIRAGE

(43) Date of publication of application: 26.06.2019
(62) Divisional of application: 13156586.3
(73) Proprietor: Steris Instrument Management Services, Inc., Birmingham, AL 35222 (US)
(72) Inventor: YE, Shusheng, Davie, FL 33328 (US); BODOR, Zoltan A., Plantation, FL 33325 (US); MCKENNA, Sean, Miami, FL 33136 (US); BODOR, Peter Pal, Pembroke Pines, FL 33029 (US)
(74) Representative: Range, Christopher William

(56) References cited:
- WO-A1-00/48505
- WO-A2-2006/105283
- JP-A- 2001 112 770
- US-A- 5 575 756
- US-A1- 2006 036 132

## Description

### Field of the Invention

The present invention is directed to a rigid endoscope, and more particularly, to a distal portion of a rigid endoscope and method of assembling same.

### Background of the Invention

The distal portion of an autoclavable rigid endoscope typically consists of an inner tubular portion, an outer tubular portion and a composite of epoxy adhesive and optical fibers that fill in spaces in between the inner and outer tubular portions. During autoclave cycles the outer tube heats up and expands while the inner tube remains cool and does not expand. The difference in expansion results in shearing stresses that cause separation and failure of the epoxy adhesive joint.

JP 2001112770 discloses a trocar outer tube.

WO2006/105283 discloses an atraumatic instrument sheath

### Summary of the Invention

The present invention is directed to an endoscope optical subassembly as described in claim 1.

According to one aspect of the invention, there is provided an endoscope distal tip including a tubular assembly including a distal end, a proximal end, an outer endoscope tube, an inner endoscope tube, and at least one connecting rib extending to and between the outer endoscope tube and the inner endoscope tube and recessed from the distal end of the tubular assembly. A plurality of optical fibers extend longitudinally within a space formed between the outer endoscope tube and the inner endoscope tube. A distal end section of the plurality of optical fibers is divided into at least two spaced apart bundles of optical fibers. A tubular member having a tapered exterior surface is provided between the inner endoscope tube and the outer endoscope tube and distally to the connecting rib for aggregating the at least two spaced apart bundles of optical fiber into a single bundle of optical fibers. The tubular member may be provided between the inner tube and the plurality of optical fibers or between the plurality of optical fibers and the outer endoscope tube. Alternatively, a curved member can be used to aggregate the at least two bundles of optical fibers positioned between the outer endoscope tube and the plurality of optical fibers and distally to the connecting rib. In each instance, the tubular member and the curved member displace a portion of the plurality of optical fibers.

According to another aspect of the invention, there is provided an endoscope distal tip including a tubular assembly including a distal end, a proximal end, an outer tube, an inner tube, at least one rib rigidly coupling the outer tube to the inner tube, and a plurality of optical fibers provided between the outer tube and the inner tube, the plurality of optical fibers having a midsection divided into at least two optical fiber bundles and a distal end section provided as a single optical fiber bundle. The single optical fiber bundle is provided within a recess formed between the distal end of the tubular assembly and a distal end of the at least one rib. The proximal end of the tubular assembly is coupled to an endoscope optical subassembly including an optical tube having key hole shaped slots formed at both ends thereof, the optical tube being coupled between an objective housing and an ocular housing. The proximal end of the tubular assembly is coupled to an endoscope illumination subassembly including an illumination tube, a scope body and a flexible diaphragm coupled between the illumination tube and the scope body.

According to yet another aspect of the invention, there is provided a method of assembling an endoscope distal tip including providing an endoscope tip tubular assembly including an outer tube rigidly coupled to an inner tube by at least one rib extending there between, and providing a plurality of optical fibers within a space formed between the outer tube and the inner tube. The plurality of optical fibers are extended longitudinally and divided by the at least one rib into at least two optical fiber bundles. Thereafter, distal end sections of the at least two optical fiber bundles are aggregated into a single optical fiber bundle by inserting an aggregating member into a recess formed between a distal end of the at least one rib and a distal end of the endoscope tip tubular assembly. The aggregating member can be provided to encircle or partially encircle the inner tube or encircle or partially encircle the single optical fiber bundle.

### Brief Description of the Drawings

FIG. 1 is a plan view of a distal end of a distal portion of a 0° rigid endoscope in accordance with a preferred embodiment of the present invention.
FIG. 2 is a sectional view of the distal portion of the 0° rigid endoscope of FIG. 1 along line A-A.
FIG. 3 is a sectional view of the distal portion of the 0° rigid endoscope of FIG. 1 along line B-B.
FIG. 4 is an exploded view of an endoscope window assembly.
FIG. 5 is a perspective view of the endoscope window assembly of FIG. 4.
FIG. 6 is a sectional view of the endoscope window assembly of FIG. 4.
FIG. 7 is a perspective view of the distal end of the distal portion of the 0° rigid endoscope of FIG. 1 illustrating the endoscope window assembly of FIG. 4.
FIG. 8 is a sectional view of the distal portion of the 0° rigid endoscope of FIG. 7.
FIG. 9 is a side plan view of the distal portion of the 0° rigid endoscope of FIG. 7 illustrating coupling of an inner tube of the distal portion to an optical tube of an endoscope optical subassembly.
FIG. 10 is a plan view of the distal end of the distal portion of the 0° rigid endoscope of FIG. 9.
FIG. 11 is a sectional view of the distal portion of the 0° rigid endoscope and optical tube of FIG. 10 along line C-C.
FIG. 12 is a perspective view of the distal end of the distal portion of the 0° rigid endoscope of FIG. 9 illustrating coupling of an outer tube of the distal portion to an illumination tube of an endoscope illumination subassembly and the arrangement of a plurality of optical fibers therein.
FIG. 13 is a plan view of the distal end of the distal portion of the 0° rigid endoscope of FIG. 12.
FIG. 14 is a perspective view of an aggregating member in accordance with a preferred embodiment of the present invention.
FIG. 15 is a plan view of a distal end of the aggregating member of FIG. 14.
FIG. 16 is a sectional view of the aggregating member of FIG. 15 along line D-D.
FIG. 17 is sectional view of the distal portion of the 0° rigid endoscope of FIG. 12 illustrating the aggregating member of FIG. 14.
FIG. 18 is a perspective view of the distal end of the distal portion of the 0° rigid endoscope of FIG. 17.
FIG. 19 is a plan view of the distal end of the distal portion of the 0° rigid endoscope of FIG. 17.
FIG. 20 is a perspective view of a distal end of a distal portion of a 30° rigid endoscope in accordance with a preferred embodiment of the present invention.
FIG. 21 is a side plan view of the distal portion of the 30° rigid endoscope of FIG. 20.
FIG. 22 is a plan view of the distal end of the distal portion of the 30° rigid endoscope of FIG. 20.
FIG. 23 is a sectional view of the distal portion of the 30° rigid endoscope of FIG. 22 along line E-E.
FIG. 24 is a side plan view of the distal portion of the 30° rigid endoscope of FIG. 20 illustrating coupling of an outer tube of the distal portion with an illumination tube of an endoscope illumination subassembly.
FIG. 25 is a plan view of the distal end of the distal portion of the 30° rigid endoscope of FIG. 24 illustrating the arrangement of a plurality of optical fibers therein.
FIG. 26 is a partially exploded view of the distal end of the distal portion of the 30° rigid endoscope of FIG. 24 illustrating an aggregating member in accordance with a preferred embodiment of the present invention.
FIG. 27 is a perspective view of the distal end of the distal portion of the 30° rigid endoscope of FIG. 26 illustrating the aggregating member of FIG. 26.
FIG. 28 is a plan view of the distal end of the distal portion of the 30° rigid endoscope of FIG. 27.
FIG. 29 is a partially exploded view of an endoscope optical tube in accordance with a preferred embodiment of the present invention coupled between an objective housing and an ocular housing.
FIG. 30 is a perspective view of the optical tube of FIG. 29.
FIG. 31 is a sectional view of an endoscope including an optical tube of an endoscope optical subassembly flexibly coupled to a scope body in accordance with a preferred embodiment of the present invention.
FIG. 32 is a detail sectional view of the flexible diaphragm of the endoscope illumination subassembly of FIG. 31 coupling a scope body to an optical tube.
FIG. 33 is a perspective view of the flexible diaphragm of FIG. 31.
FIG. 34 is a sectional view of the flexible diaphragm of FIG. 33.
FIG. 35 is a perspective view of an alternatively designed flexible diaphragm.
FIG. 36 is a sectional view of the flexible diaphragm of FIG. 35.
FIG. 37 is a sectional view of the endoscope of FIG. 32 including an illumination subassembly coupled to an endoscope eyepiece of an endoscope optical subassembly in accordance with a preferred embodiment of the present invention.

### Detailed Description of Presently Preferred Embodiments

The present invention is directed an endoscope that is capable of withstanding harsh conditions associated with medical equipment cleaning and sterilization, particularly, the high temperatures and pressures associated with autoclaving. More particularly, the present invention is directed to a distal portion of a rigid endoscope including an inner tube and an outer tube rigidly fixed thereto using permanent means and various methods for assembling endoscopes including methods for installing optical fibers and assembling endoscope subassemblies.

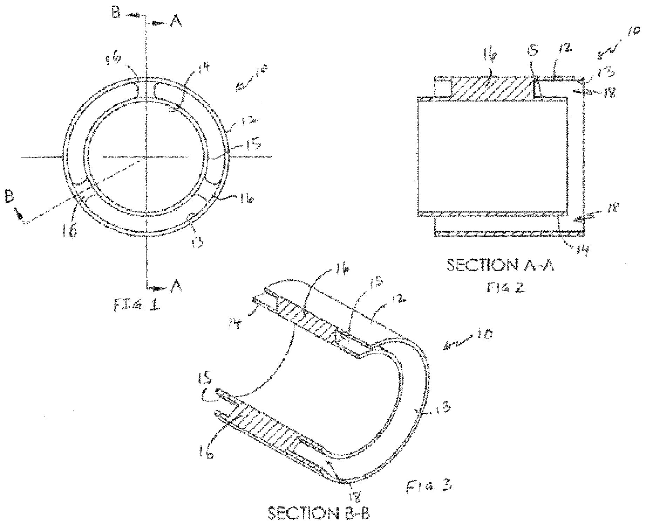

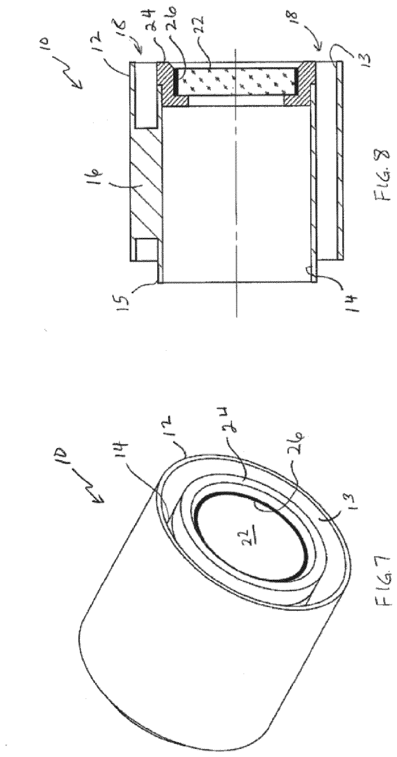

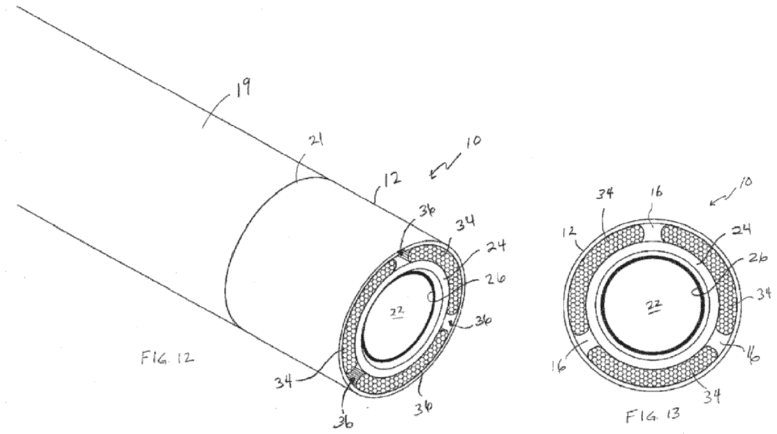

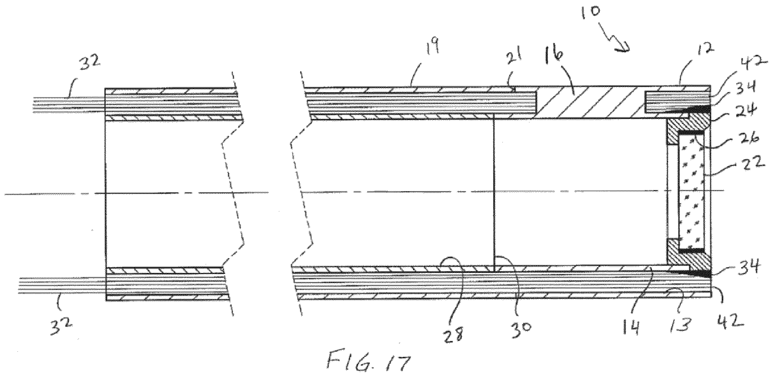

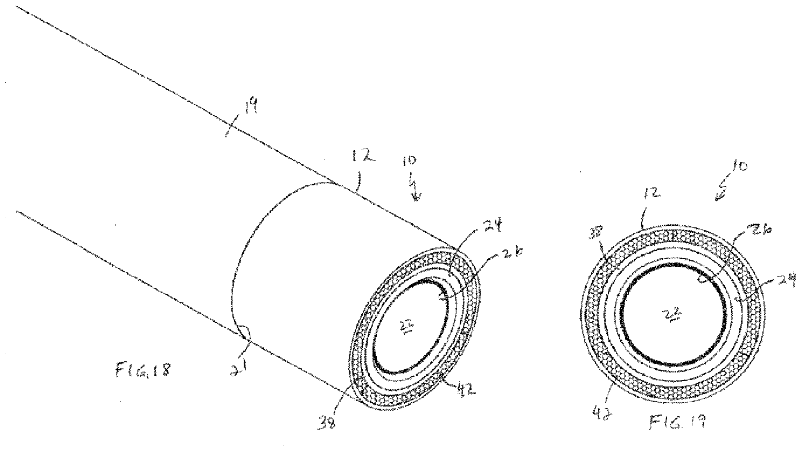

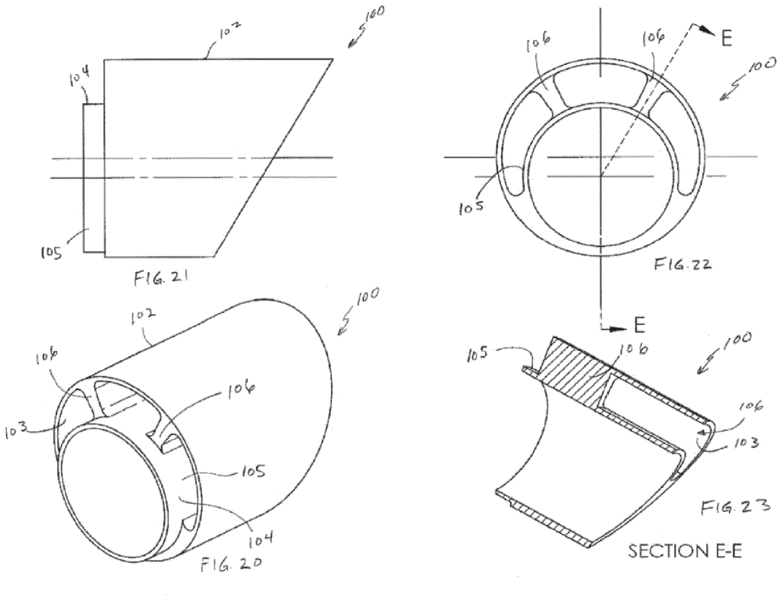

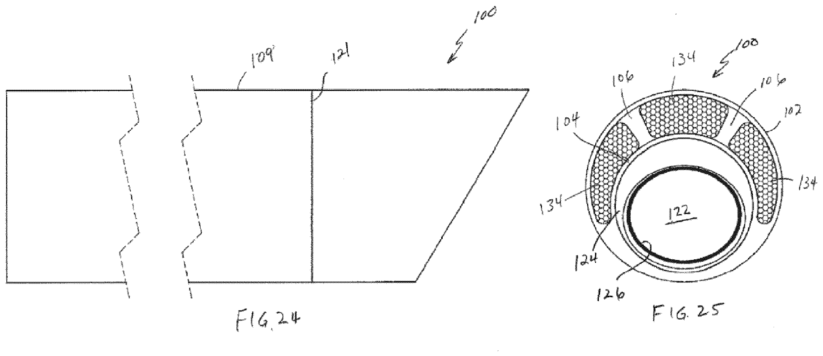

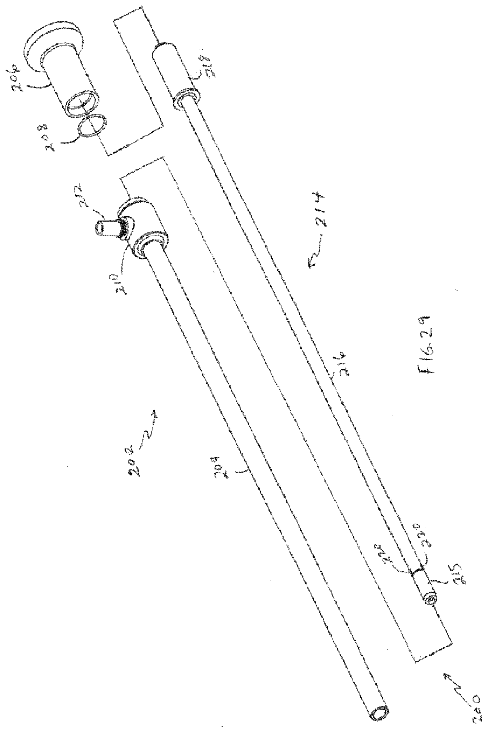

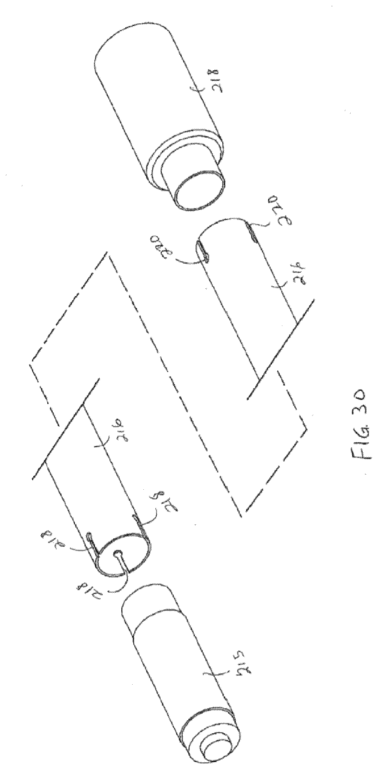

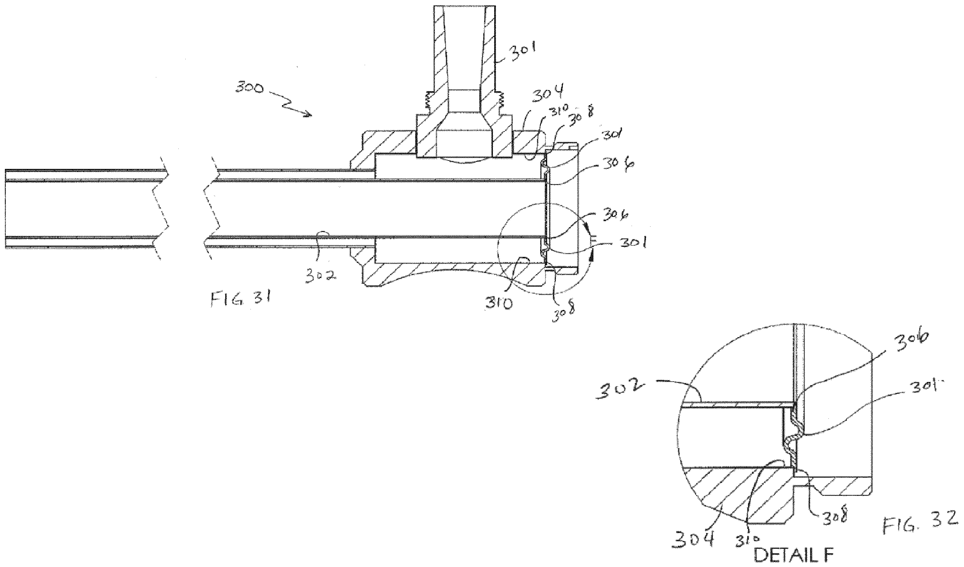

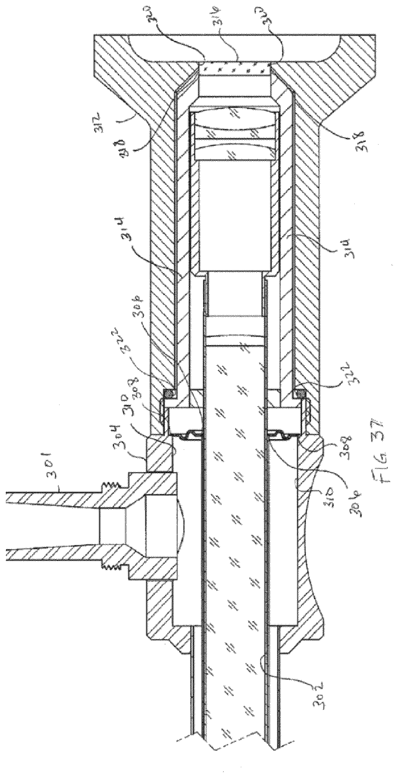

Referring to the drawings, FIGS. 1 through 19 illustrate a distal portion of a 0° rigid endoscope in accordance with a preferred embodiment of the present invention. FIGS. 20 through 28 illustrate a distal portion of a 30° rigid endoscope in accordance with a preferred embodiment of the present invention. FIGS. 29 and 30 illustrate an optical tube in accordance with a preferred embodiment of the present invention for use in an endoscope optical subassembly. FIGS. 31 through 36 illustrate a means of flexibly coupling an endoscope optical tube of an endoscope optical subassembly to a scope body in accordance with a preferred embodiment of the present invention. FIG. 37 illustrates an endoscope illumination subassembly coupled to an endoscope eyepiece of an endoscope optical subassembly in accordance with a preferred embodiment of the present invention.

Referring to the drawings, FIGS. 1 through 19, there is depicted a distal portion 10 of a 0° rigid endoscope including an outer tube 12, an inner tube 14, and three ribs 16 which extend to and between outer tube 12 and inner tube 14 for permanently and rigidly coupling tubes 12 and 14 together. Outer tube 12 is cylindrically-shaped having a inner surface 13. Inner tube 14 is cylindrically-shaped, having an outer surface 15. Inner tube 14 is concentrically arranged within outer tube 12 and recessed within a distal end thereof, while extending outwardly from a proximal end of outer tube 12. Ribs 16 are equidistantly spaced about inner tube 14 and extend from outer surface 15 of inner tube 14 to inner surface 13 of outer tube 12. Ribs 16 have a rectangular cross-section and are recessed from the distal end of outer tube 12 thereby forming a recess 18. It is contemplated that ribs 16 can be flush with the distal end of outer tube 12. Distal portion 10 can be manufactured using known machining techniques such as milling or wire electric discharge machining (EDM).

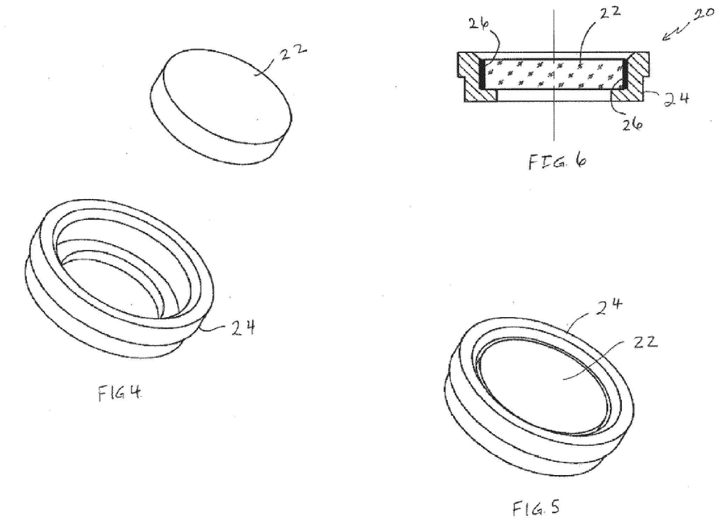

Referring to FIGS. 4 through 10, there is depicted a window assembly 20 for insertion into the distal end of distal portion 10. Window assembly 20 includes a window 22 constructed of sapphire or other suitable material that is brazed directly to a window seat 24 made of titanium or similar material along a joint 26. Window assembly 20 is inserted into the distal end of distal portion 10, and window seat 24 is directly coupled to inner tube 14 by soldering or brazing window seat 24 to the distal end of inner tube 14. Preferably, the solder or braze temperature is less than the temperature used to braze window 22 within window seat 24.

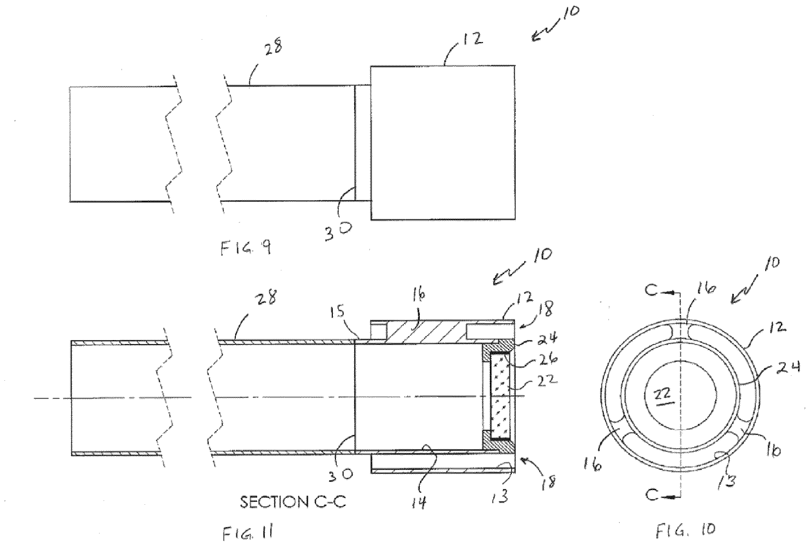

Referring to FIGS. 9 through 11, following insertion of window assembly 20 into distal portion 10, an endoscope optical tube 28 of an endoscope optical tube assembly is welded to the proximal end of inner tube 14 along a joint 30. A plurality of optical fibers 32 is then extended through the space formed between inner surface 13 of outer tube 12 and outer surface 15 of inner tube 14 from the proximal end of distal portion 10 to be flush with the distal end of outer tube 12. As plurality of optical fibers 32 are inserted into distal portion 10, the fibers are diverted around and to each side of each of ribs 16 thereby dividing the midsection and distal end of plurality of optical fibers 32 into three optical fiber bundles 34 separated by gaps 36. The width of each of gaps 36 is substantially equal to the width of each of ribs 16. Thereafter, an illumination tube 19 of an endoscope illumination subassembly is laser welded to a proximal end of outer tube 12 along joint 21 with plurality of optical fibers 32 extending there through.

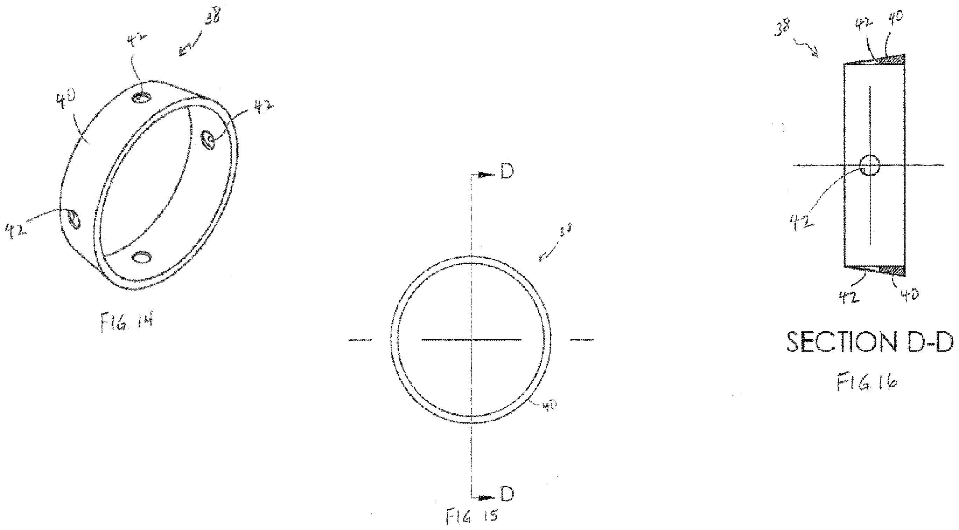

Referring FIGS. 14 through 16, there is depicted an aggregating member 38 for aggregating the three optical fiber bundles 34 at the distal end of plurality of optical fibers 32 into a single optical fiber bundle in order to provide even lighting at the tip of distal portion 10 when in use. Aggregating member 38 is cylindrically-shaped having a constant inner diameter that matches the outer diameter of inner tube 14, a tapered exterior surface 40 thereby providing the sidewall thereof to have a variable thickness, and a plurality of glue holes 42 through the sidewall. To aggregate the three optical fiber bundles 34 into a single optical fiber bundle, aggregating member 38 is axially aligned with inner tube 14 with thin end of the sidewall of aggregating member 38 facing distal portion 10. Aggregating member 38 is then pressed onto inner tube 14 and into recess 18 while epoxy is applied to the three optical fiber bundles 34. As aggregating 38 member is pressed onto inner tube 14, tapered exterior surface 40 rides against the interior surfaces of the three optical fiber bundles 34. As the thicker potion of the aggregating member 38 sidewall advances along the three optical fiber bundles 34, the optical fibers are displaced by aggregating member 38 and forced into gaps 36. In so doing, the three optical fiber bundles 34 are merged into a single optical fiber bundle 44.

Referring to the drawings, FIGS. 21 through 28, there is depicted a distal portion 100 of a 30° rigid endoscope including an outer tube 102, an inner tube 104, and two ribs 106 which extend to and between outer tube 102 and inner tube 104 for permanently and rigidly coupling tubes 102 and 104 together. Outer tube 102 is cylindrically-shaped having a inner surface 103. Inner tube 104 is cylindrically-shaped, having an outer surface 105. Inner tube 104 is provided within outer tube 102 is an offset arrangement so that inner tube 104 and outer tube 102 share a sidewall portion 107. The distal end of inner tube 104 is flush with the distal end of outer tube 102, while the proximal end of inner tube 104 extends outwardly from a proximal end of outer tube 102. Ribs 106 are spaced about inner tube 104 and extend from outer surface 105 of inner tube 104 to inner surface 103 of outer tube 102. Ribs 106 have a rectangular cross-section and are recessed from the distal end of outer tube 102 thereby forming a recess 108. Distal portion 100 can be manufactured using known machining techniques such as milling or wire EDM

As in distal portion 10, distal portion 100 includes a window assembly for insertion into the distal end of distal portion 100. The window assembly includes a window 122 that is brazed directly to a window seat 124 along a joint 126. The window assembly is inserted into the distal end of distal portion 100, and window seat 124 is directly coupled to inner tube 104 by soldering or brazing window seat 124 to the distal end of inner tube 104. Further, as in distal portion 10, following insertion of the window assembly into distal portion 100, an endoscope optical tube of an endoscope optical tube assembly is laser welded to the proximal end of inner tube 14 along a joint. A plurality of optical fibers is then extended through the space formed between inner surface 103 of outer tube 102 and outer surface 105 of inner tube 104 from the proximal end of distal portion 100 to be flush with the distal end of outer tube 102. As the plurality of optical fibers are inserted into distal portion 100, the fibers are diverted around and to each side of each of ribs 106 thereby dividing the midsection and distal end of the plurality of optical fibers into three optical fiber bundles 134 separated by gaps 136. The width of each of gaps 136 is substantially equal to the width of each of ribs 106.

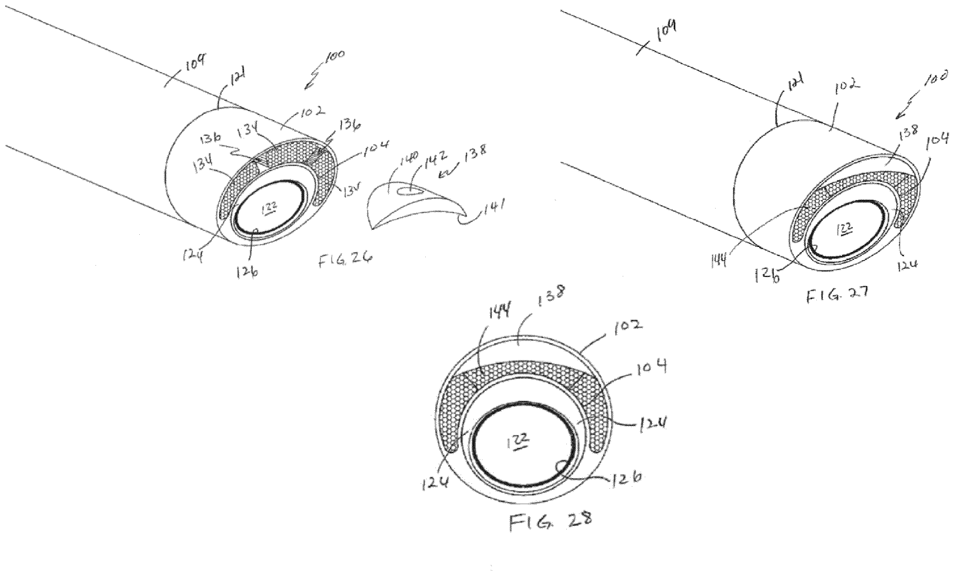

Referring FIGS. 26 through 28, there is depicted an aggregating member 138 for aggregating the three optical fiber bundles 134 at the distal end of the plurality of optical fibers into a single optical fiber bundle in order to provide even lighting at the tipoff distal portion 100 when in use. Aggregating member 138 is wedge-shaped having a curved exterior surface 140 matching the curvature of inner surface 103 of outer tube 102, a curved interior surface 141 having a less pronounced curve than exterior surface 140 thereby providing aggregate member 138 a crescent-shaped cross-section, a tapered sidewall thickness having a thicker distal side than proximal side, and a glue hole 142. To aggregate the three optical fiber bundles 134 into a single optical fiber bundle, aggregating member 138 is aligned with recess 106 with thin side of the sidewall of aggregating member 138 facing distal portion 100. Aggregating member 138 is then pressed into recess 106 with curved exterior surface 140 pressed against inner surface 103 of outer tube 102 while epoxy is applied to the three optical fiber bundles 134. As aggregating member 138 is pressed into recess 106, interior surface 141 rides against the exterior surfaces of the three optical fiber bundles 134. As the thicker potion of the aggregating member 138 sidewall advances along the three optical fiber bundles 134, the optical fibers are displaced by aggregating member 138 and forced into gaps 136. In so doing, the three optical fiber bundles 134 are merged into a single optical fiber bundle 144.

Referring to FIGS. 29 and 30, there is a depicted an endoscope illumination and optical assembly 200 in accordance with a preferred embodiment of the present invention. Assembly 200 includes an illumination subassembly 202 having an illumination tube 204, an eye piece 206, a sealing element 208, a scope body 210 and a light cone 212. Assembly 200 further includes an optical subassembly 214 including an optical tube 216, an objective housing 215, and an ocular housing 218, all of which are concentrically contained within illumination subassembly 202. Each end of optical tube 216 includes a plurality of keyhole shaped slots 220 which may be cut by a laser of other machining means. Objective housing 215 is lightly press-fitted into the distal end of optical tube 216. The elastic properties imparted to the distal end of optical tube 216 by the inclusion of key hole shaped slots 220 are used to maintain objective housing 215 in place temporarily to allow minor adjustments before housing 215 is glued in place. Slots 220 also provide larger surface areas for the glue to seep in between optical tube 216 and objective housing 215 thereby improving adherence of the joint there between. Similarly, the elastic properties imparted to the proximal end of optical tube 216 by the inclusion of key hole shaped slots 220 are used to maintain ocular housing 218 in place temporarily to allow minor adjustments before housing 218 is glued in place. Slots 220 also provide larger surface areas for the glue to seep in between optical tube 216 and ocular housing 218 thereby improving adherence of the joint there between. Optical system components such as rod lenses and spacer are loaded into optical tube 216.

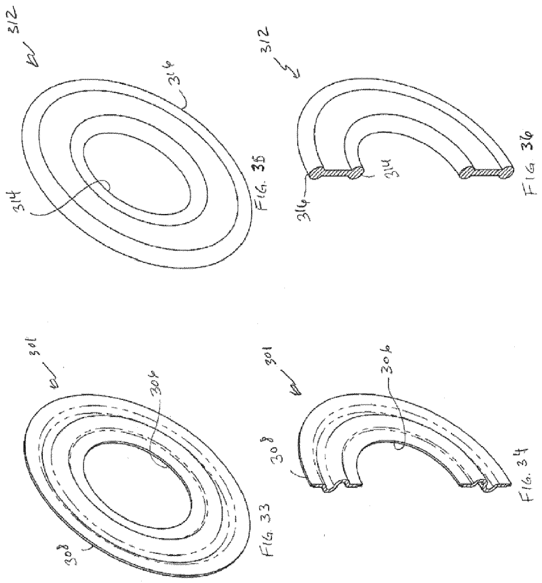

Referring to FIGS. 31 and 36, there is depicted an endoscope 300 including a flexible diaphragm 301 for coupling an endoscope optical tube 302 of an endoscope optical subassembly to a scope body 304 having a light cone 301. Diaphragm 301 permits deflection of optical tube 302 in the axial direction and thus relieves thermal expansion and stresses in optical tube 302 during sterilization. As depicted in FIGS. 34 and 36, diaphragm 300 can be provided as a wavy steel washer having inner circular edge 306 that is welded about and to the proximal end of optical tube 302 and its outer circular edge 308 welded to an interior surface 310 of scope body 304. Alternatively, diaphragm 301 may be a silicone rubber washer 312 with a dumbbell shaped cross-section as depicted in FIGS. 36 and 38. An inner circular edge 314 of diaphragm 312 can be slipped over the proximal end of optical tube 302 and sandwiched by thrust nuts. An outer edge 316 of diaphragm 312 can be similarly attached to scope body 304.

Referring to FIG. 37, there is depicted endoscope 300 illustrating an eyepiece housing 312 coupled to endoscope optical tube 302 and scope body 304. Eyepiece housing 312 contains a window housing 314 arranged concentrically therein. At the proximal end of eyepiece housing 314, a window 316 is soldered to window housing 314 at joint 318 and eyepiece housing 312 at joint 320. At the distal end of eyepiece housing 312, window housing 314 is welded to optical tube 302 and the proximal end of scope body 304 is coupled to and enclosed by eyepiece housing 312. A sealing element 322 is provided between scope body 304 and eyepiece housing 312.

As will be apparent to one skilled in the art, various modifications can be made within the scope of the aforesaid description. Such modifications being within the ability of one skilled in the art form a part of the present invention and are embraced by the claims below.

## Claims

1. An endoscope optical subassembly comprising:
an optical tube (302) containing one or more optical lens components,
a scope body (304), **characterized in that** the endoscope optical subassembly further comprises a flexible diaphragm (301) coupled to and between the optical tube (302) and the scope body (304), and an illumination tube through which the optical tube (302) extends,
wherein the illumination tube is coupled to the scope body (304) and axially spaced-apart from the flexible diaphragm (301).

2. The endoscope optical subassembly according to claim 1 wherein the optical tube (302) is deflectable in an axial direction relative to the scope body (304), due to the flexible diaphragm (301) that is configured to couple the optical tube (302) to the scope body (304).

3. The endoscope optical subassembly according to claim 1 wherein the illumination tube houses optical fibres configured to transmit light.

4. The endoscope optical subassembly according to claim 3 wherein the optical tube (302) is movable axially along a longitudinal axis thereof relative to the illumination tube.

5. The endoscope optical subassembly according to any preceding claims further comprising a light cone and an eyepiece assembly (312) coupled directly to the scope body (304).

6. The endoscope optical subassembly according to any preceding claim wherein the diaphragm (301) is a wavy metal washer.

7. The endoscope optical subassembly according to any of claims 1 to 5 wherein the diaphragm is constructed of rubber.

8. The endoscope optical assembly according to any preceding claim wherein the diaphragm (301) has an inner edge (306) coupled to and encircling a proximal end of the optical tube (302) and an outer edge (308) coupled to the scope body (304).

9. The endoscope optical assembly according to any preceding claim wherein the diaphragm (301) has a dumbbell-shaped cross-section.

10. The endoscope optical subassembly according to any preceding claim wherein the diaphragm (301) includes a substantially S-shaped cross-section portion.

11. An endoscope comprising an endoscope optical subassembly as claimed in any preceding claim.

12. The endoscope according to claim 11, wherein the flexible diaphragm (301) has an inner edge (306) coupled to the said optical tube (302).

13. The endoscope according to claim 11 or claim 12, wherein the flexible diaphragm (301) has an outer edge (308) coupled to the scope body (304).

14. The endoscope according to any of claims 11, 12 and 13 wherein the optical tube (302) is movable axially along a longitudinal axis of the outer illumination tube.

15. The endoscope according to any of claims 11 to 14, wherein the flexible diaphragm (301) is welded directly to the optical tube (302) and directly to the scope body (304) and wherein the flexible diaphragm is a wavy steel washer having an inner circular edge.

## Patentansprüche

1. Optische Unterbaugruppe eines Endoskops, umfassend:
eine optische Röhre (302), die eine oder mehrere optische Linsenkomponenten enthält,
einen Endoskopkörper (304), **dadurch gekennzeichnet, dass** die optische Unterbaugruppe des Endoskops ferner eine flexible Membran (301) umfasst, die mit der optischen Röhre (302) und dem Endoskopkörper (304) gekoppelt und dazwischen angeordnet ist, sowie eine Beleuchtungsröhre, durch die sich die optische Röhre (302) erstreckt,
wobei die Beleuchtungsröhre mit dem Endoskopkörper (304) gekoppelt und axial beabstandet von der flexiblen Membran (301) ist.

2. Optische Unterbaugruppe eines Endoskops nach Anspruch 1, wobei die optische Röhre (302) in einer axialen Richtung relativ zu dem Endoskopkörper (304) auslenkbar ist, aufgrund der flexiblen Membran (301), die dazu konfiguriert ist, die optische Röhre (302) mit dem Endoskopkörper (304) zu koppeln.

3. Optische Unterbaugruppe eines Endoskops nach Anspruch 1, **dadurch gekennzeichnet, dass** die Beleuchtungsröhre Glasfaserleiter zum Übertragen von Licht enthält.

4. Optische Unterbaugruppe eines Endoskop nach Anspruch 3, **dadurch gekennzeichnet, dass** die optische Röhre (302) axial entlang ihrer Längsachse relativ zu der Beleuchtungsröhre bewegbar ist.

5. Optische Unterbaugruppe eines Endoskops nach einem der vorhergehenden Ansprüche, ferner umfassend einen Lichtkegel und eine Okularbaugruppe (312), die direkt mit dem Endoskopkörper (304) gekoppelt ist.

6. Optische Unterbaugruppe eines Endoskops nach einem der vorhergehenden Ansprüche, wobei die Membran (301) eine gewellte Metallscheibe ist.

7. Optische Unterbaugruppe eines Endoskops nach einem der Ansprüche 1 bis 5, wobei die Membran aus Gummi gefertigt ist.

8. Optische Unterbaugruppe eines Endoskops nach einem der vorhergehenden Ansprüche, wobei die Membran (301) einen inneren Rand (306), der mit einem proximalen Ende der optischen Röhre (302) gekoppelt ist und diese umgibt, und einen äußeren Rand (308), der mit dem Endoskopkörper (304) gekoppelt ist, aufweist.

9. Optische Unterbaugruppe eines Endoskops nach einem der vorhergehenden Ansprüche, wobei die Membran (301) einen hantelförmigen Querschnitt aufweist.

10. Optische Unterbaugruppe eines Endoskops nach einem der vorhergehenden Ansprüche, wobei die Membran (301) einen im Wesentlichen S-förmigen Querschnittsabschnitt aufweist.

11. Endoskop, umfassend eine optische Unterbaugruppe eines Endoskops nach einem der vorhergehenden Ansprüche.

12. Endoskop nach Anspruch 11, wobei die flexible Membran (301) einen inneren Rand (306) aufweist, der mit der optischen Röhre (302) gekoppelt ist.

13. Endoskop nach Anspruch 11 oder Anspruch 12, wobei die flexible Membran (301) einen äußeren Rand (308) aufweist, der mit dem Endoskopkörper (304) gekoppelt ist.

14. Endoskop nach einem der Ansprüche 11, 12 und 13, wobei die optische Röhre (302) axial entlang einer Längsachse der äußeren Beleuchtungsröhre bewegbar ist.

15. Endoskop nach einem der Ansprüche 11 bis 14, wobei die flexible Membran (301) direkt mit der optischen Röhre (302) und direkt mit dem Endoskopkörper (304) verschweißt ist und wobei die flexible Membran eine wellenförmige Stahlscheibe mit einem inneren kreisförmigen Rand ist.

## Revendications

1. Sous-ensemble optique d'endoscope comprenant :
un tube optique (302) contenant un ou plusieurs composants de lentille optique,
un corps d'endoscope (304), **caractérisé en ce que** le sous-ensemble optique d'endoscope comprend également un diaphragme souple (301) couplé au tube optique (302) et au corps d'endoscope (304) et entre ceux-ci, et un tube d'éclairage traversé par le tube optique (302),
dans lequel le tube d'éclairage est couplé au corps d'endoscope (304) et axialement espacé du diaphragme souple (301) .

2. Sous-ensemble optique d'endoscope selon la revendication 1, dans lequel le tube optique (302) est déplaçable dans une direction axiale par rapport au corps d'endoscope (304), grâce au diaphragme souple (301) qui est configuré pour coupler le tube optique (302) au corps d'endoscope (304).

3. Sous-ensemble optique d'endoscope selon la revendication 1, dans lequel le tube d'éclairage loge des fibres optiques configurées pour transmettre la lumière.

4. Sous-ensemble optique d'endoscope selon la revendication 3, **caractérisé en ce que** le tube optique (302) est mobile axialement le long d'un axe longitudinal de celui-ci par rapport au tube d'éclairage.

5. Sous-ensemble optique d'endoscope selon l'une quelconque des revendications précédentes, comprenant également un cône de lumière et un ensemble oculaire (312) couplé directement au corps d'endoscope (304).

6. Sous-ensemble optique d'endoscope selon une quelconque revendication précédente, dans lequel le diaphragme (301) est une rondelle métallique ondulée.

7. Sous-ensemble optique d'endoscope selon l'une quelconque des revendications 1 à 5, dans lequel le diaphragme est constitué de caoutchouc.

8. Ensemble optique d'endoscope selon une quelconque revendication précédente, dans lequel le diaphragme (301) a un bord interne (306) couplé à et entourant une extrémité proximale du tube optique (302) et un bord externe (308) couplé au corps d'endoscope (304).

9. Ensemble optique d'endoscope selon une quelconque revendication précédente, dans lequel le diaphragme (301) a une section transversale en forme d'haltère.

10. Sous-ensemble optique d'endoscope selon une quelconque revendication précédente, dans lequel le diaphragme (301) comporte une partie de section sensiblement en forme de S.

11. Endoscope comprenant un sous-ensemble optique d'endoscope selon une quelconque revendication précédente.

12. Endoscope selon la revendication 11, dans lequel le diaphragme souple (301) a un bord interne (306) couplé audit tube optique (302).

13. Endoscope selon l'une quelconque des revendications 11 à 12, dans lequel le diaphragme souple (301) a un bord externe (308) couplé au corps d'endoscope (304).

14. Endoscope selon l'une quelconque des revendications 11, 12 et 13, dans lequel le tube optique (302) est mobile axialement selon un axe longitudinal du tube d'éclairage externe.

15. Endoscope selon l'une quelconque des revendications 11 à 14, dans lequel le diaphragme souple (301) est soudé directement sur le tube optique (302) et directement sur le corps d'endoscope (304) et dans lequel le diaphragme souple est une rondelle d'acier ondulée à bord circulaire interne.
